# FASCICULE DE BREVET EUROPEEN

(11) **EP 0 598 458 B1**
(45) Date de publication et mention de la délivrance du brevet: **20.09.1995**
(21) Numéro de dépôt: 93203226.1
(22) Date de dépôt: 18.11.1993
(51) Int. Cl.: A01K 67/033, A01K 47/00

(54) **Réfrigérateur pour la prévention de hautes températures dans des nichoirs pour colonies de faux-bourdons**
Kühlschrank zur Vermeidung von Hochtemperatur in Nistkasten für Hummelkolonien
Cooling installation preventing high temperatures in nestboxes for bumble bee colonies

(30) Priorité: 17.11.1992 BE 9200994
(43) Date de publication de la demande: 25.05.1994
(73) Titulaire: De Vleeschouwer, Luc, B-2550 Waarloos (BE)
(72) Inventeur: De Vleeschouwer, Luc, B-2550 Waarloos (BE)
(74) Mandataire: Ottelohe, Jozef René

(56) Documents cités:
- DE-A- 3 718 387
- DATABASE WPI Section PQ, Week 8144, Derwent Publications Ltd., London, GB; Class P14, AN 81-L2322D & DD-A-149 899 (SIEMON) 5 Aoüt 1981
- DATABASE WPI Section PQ, Week 8919, Derwent Publications Ltd., London, GB; Class P14, AN 89-143852 & SU-A-1 436 959 (RYBKIN) 15 Novembre 1988

## Description

L'invention concerne un réfrigérateur simple et bon marché, fonctionnant économiquement, dans lequel on place des nichoirs pour faux-bourdons et dans lequel il est assuré que la température dans les nichoirs ne monte pas trop haute et qu'elle reste à peu près constante dans tous les nichoirs.

Le logement de nichoirs dans une serre est connu. Un inconvénient important est toutefois que très souvent la température dans la serre et par conséquent dans les nichoirs monte au-delà de 32°C, par suite de quoi le couvain de faux-bourdons dans les nichoirs meurt très vite et que dans les nichoirs en même temps des ouvrières ne sont plus produites, mais seulement des faux-bourdons improductifs, de sorte que plus aucune fécondation n'a lieu dans le nichoir.

Pour remédier à cet inconvénient, selon la caractéristique principale de l'invention un réfrigérateur est réalisé qui est pourvu de parois et d'un fond et d'un couvercle en matière calorifuge, d'une conduite d'eau pour le refroidissement de l'espace intérieur du casier, conduite dont la sortie est raccordée à une installation d'arrosage du toit ou d'alimentation goutte à goutte d'une serre, d'un robinet d'arrêt monté sur la conduite d'eau, de plusieurs tablettes en matière calorifuge qui divisent le casier en plusieurs compartiments séparés égaux dans lesquels sont placés les nichoirs pour les faux-bourdons, d'une plaque frontale commune en matière calorifuge qui ferme le casier sur le devant et dans laquelle pour chaque compartiment sont prévus une ouverture de vol et des trous d'aération pour laisser passer les faux-bourdons et l'air, et d'une cloison amovible en matière calorifuge pour fermer l'arrière-côté de chaque compartiment.

Ce casier est fixé contre les montants de la serre et raccordé à une conduite d'eau. Abstraction faite de ce que la température ne peut plus monter au-delà de 32°C grâce au refroidissement régulier, grâce aux compartiments séparés du casier la chaleur ne peut plus parvenir dans les compartiments supérieurs, de sorte que dans tous les compartiments règne une température à peu près constante. Un autre avantage important est que pour le refroidissement du casier il est fait usage de l'eau de l'installation d'arrosage du toit de la serre dans laquelle le casier est placé ou de l'installation d'alimentation goutte à goutte avec compte-gouttes donnant 24 L/h.

A titre d'exemple, sans caractère limitatif, suit ci-dessous une description détaillée d'une forme choisie d'exécution du réfrigérateur conforme à l'invention. Cette description renvoie aux dessins ci-joints, dans lesquels :

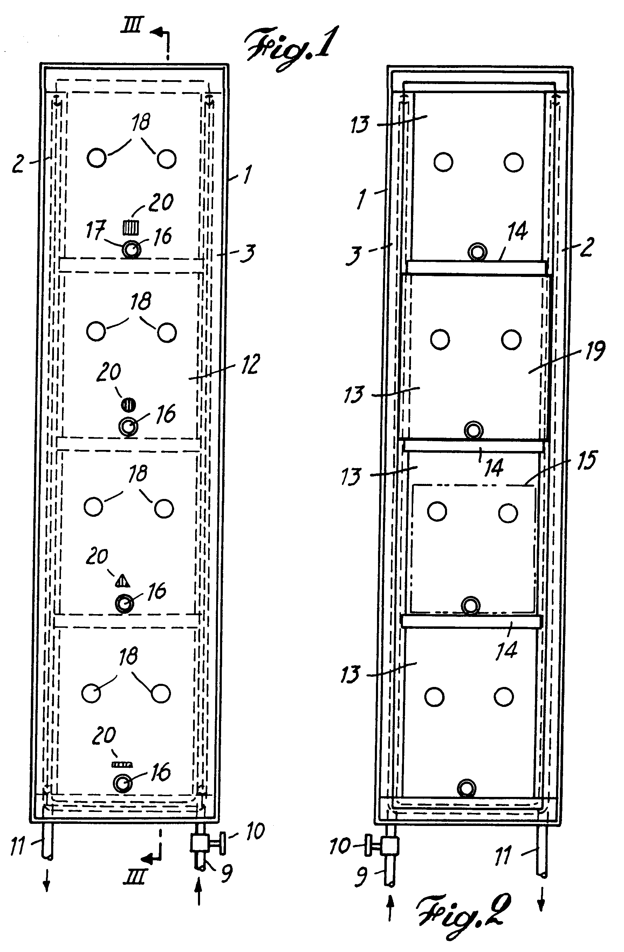

la fig. 1 représente une élévation de devant du réfrigérateur;

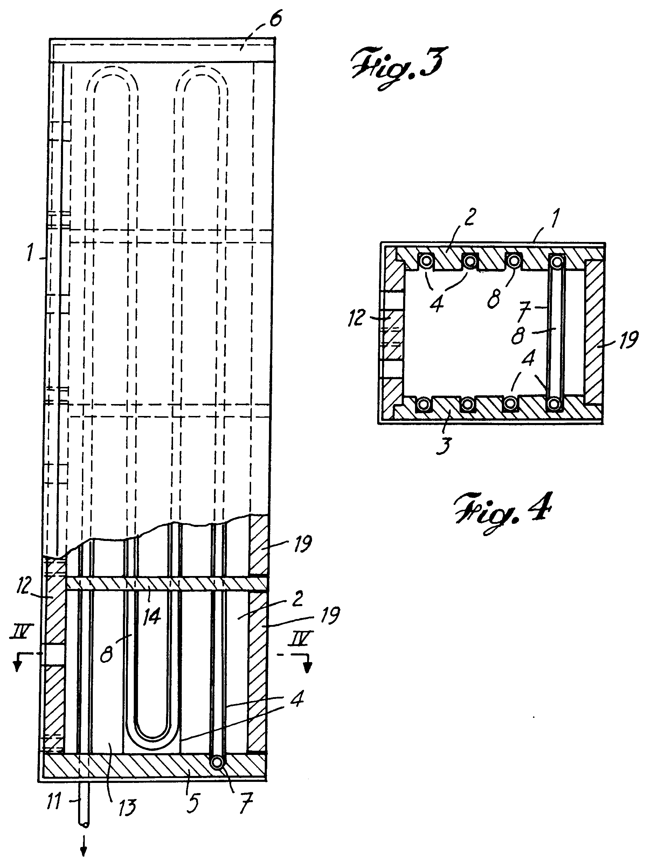

la fig. 2 représente une élévation de derrière de celui-ci, dont un compartiment est fermé par une cloison et dont les autres compartiments sont laissés ouverts pour plus de clarté'
la fig. 3 représente une coupe longitudinale du casier suivant la ligne III-III de la fig. 1;
la fig. 4 représente une coupe transversale du casier suivant la ligne IV-IV de la fig. 3.

Dans ces figures, on constate que le casier 1 est pourvu de deux parois 2-3 en matière calorifuge posées l'une en face de l'autre, comme le soi-disant "Tempex" et dans lesquelles sont prévues, le long du côté intérieur, des rainures 4. Le fond 5 et le couvercle 6 du casier sont également fabriqués en matière calorifuge, par exemple le "polystyrène", et dans le fond une rainure 7 est prévue. Dans les rainures 4-7 des parois et du fond du casier est placée une conduite d'eau 8 en PVC flexible, dont l'entrée 9 est pourvue d'un robinet d'arrêt 10 et est raccordée à une conduite d'eau existante. La sortie 11 est raccordée à une installation existante d'arrosage du toit d'une serre dans laquelle le casier est placé. De cette manière, pour le refroidissement du casier il n'est pas fait usage d'eau supplémentaire, mais de l'eau de l'installation d'arrosage du toit de la serre, et la température dans les nichoirs peut être maintenue au-dessous de 32°C sans qu'il en résulte des frais supplémentaires. La face frontale du réfrigérateur 1 est couverte d'une plaque frontale 12 faite également d'une matière calorifuge comme le polystyrène. Le casier est divisé en compartiments 13 au moyen de tablettes 14 en matière calorifuge comme le polystyrène, dans lesquels les nichoirs 15 pour les faux-bourdons sont logés séparément les uns des autres. Grâce à cela, on obtient que la chaleur ne peut pas monter dans les compartiments supérieurs et que la température reste à peu près égale dans tous les compartiments. Pour chaque compartiment, dans la plaque frontale une ouverture de vol 16 est aménagée, dans laquelle est introduit un petit tube de protection 17, de sorte que les faux-bourdons peuvent voler sans empêchement et sûrement dans et hors des nichoirs. De même, pour chaque compartiment, deux trous d'aération 18 sont prévus dans la plaque frontale. L'arrière-côté de chaque compartiment 13 est fermé par une cloison amovible 19 en matière calorifuge comme le polystyrène. Pour chaque cloison sont prévus des moyens (non représentés) de fixation des cloisons. Au-dessus de chaque ouverture de vol est disposé un signe 20 différent pour l'identification des nichoirs.

Il va de soi que le robinet d'arrêt 10 peut être commandé par un mécanisme de commande à thermostat placé dans le casier ou par ordinateur.

De même, il va de soi que n'importe quelle matière isolante peut être utilisée et que le casier peut avoir n'importe quelles dimensions et formes et peut être divisé en un nombre quelconque de compartiments.

## Revendications

1. Réfrigérateur pour la prévention de hautes températures dans des nichoirs pour colonies de faux-bourdons, caractérisé par le fait que le réfrigérateur comprend un casier (1) qui est pourvu de parois (2-3), d'un fond (5) et d'un couvercle (6) en matière calorifuge, d'une conduite d'eau (8) pour le refroidissement des espaces intérieurs du casier, conduite d'eau dont la sortie (11) est raccordée à une installation d'arrosage du toit d'une serre ou d'alimentation goutte à goutte d'une serre, d'un robinet d'arrêt (10) monté sur la conduite d'eau, de plusieurs tablettes (14) en matière calorifuge, qui divisent le casier en plusieurs compartiments (13) égaux séparés, dans lesquels les nichoirs (15) pour les faux-bourdons sont placés, d'une plaque frontale commune (12) en matière calorifuge qui ferme le casier sur le devant et dans laquelle pour chaque compartiment une ouverture de vol (16) et des trous d'aération (18) sont prévus pour laisser passer les faux-bourdons et l'air, et d'une cloison (19) amovible en matière calorifuge pour la fermeture de l'arrière-côté de chaque compartiment (13).

2. Réfrigérateur conforme à la revendication 1, caractérisé par le fait que dans la surface intérieure des parois latérales (2-3) et du fond (5) du casier sont prévues des rainures (4-7) dans lesquelles la conduite d'eau (8) est logée.

3. Réfrigérateur conforme à la revendication 1 , caractérisé par le fait que la matière calorifuge est constituée de polystyrène et le soi-disant "Tempex" et que la conduite d'eau (8) est fabriqué en PVC flexible.

4. Réfrigérateur conforme à la revendication 1, caractérisé par le fait que le robinet d'arrêt (20) est commandé par un mécanisme de commande à thermostat logé dans le casier.

5. Réfrigérateur conforme à la revendication 1, caractérisé par le fait que dans chaque ouverture de vol (16) un petit tube (17) de protection uni est monté pour la prévention de blessures aux faux-bourdons lors de leurs vols d'entrée et de sortie.

6. Réfrigérateur conforme à la revendication 1, caractérisé par le fait qu'entre le casier (1) et chaque cloison (19) des moyens de fixation sont prévus pour la fixation de chaque cloison.

## Claims

1. Cooling device for the prevention of high temperatures in nesting boxes for colonies of drones, characterized by the fact that the cooling device comprises a cabinet (1) provided with walls (2-3), a bottom (5) and a cover (6) in an insulating material, a water pipe (8) for cooling the internal spaces of the cabinet, the outlet (11) of which water pipe is connected to a spray installation of the roof of a greenhouse, or a drip feed supply of a greenhouse, a stopcock (10) fitted on the water pipe, various shelves in insulating material, which divide the cabinet into separate equal compartments (13), in which the nesting boxes (15) for the drones are placed, a common front panel (12) in an insulating material which shuts the cabinet at the front and in which for each compartment a flight hole (16) and ventilation holes (18) are provided to permit the passage of the drones and air, and a removable panel (19) in insulating material for closing the rear side of each compartment (13).

2. Cooling device in accordance with claim 1 , characterized by the fact that the internal surface of the side walls (2-3) and the bottom (5) of the cabinet are provided with grooves (4-7) in which the water pipe (8) is located.

3. Cooling device in accordance with claim 1 , characterized by the fact that the insulating material consits of polystyrene and the material called "Tempex" and the water pipe (8) is made of PVC.

4. Cooling device in accordance with claim 1 , characterized by the fact that the stopcock (20) is controlled by a thermostatic control device located in the cabinet.

5. Cooling device in accordance with claim 1, characterized by the fact that a little smooth protective tube (17) is fitted in each flight opening (16) in order to protect the drones from injury when flying in and out.

6. Cooling device in accordance with claim 1, characterized by the fact that fastening means are provided between the cabinet (1) and each panel (19) for fastening each panel.

## Patentansprüche

1. Kühlschrank zur Vermeidung hoher Temperaturen in den Nischen für Drohnenkolonien, dadurch gekennzeichnet, dass der Kühlschrank aus einem Kasten (1) besteht mit Seitenwänden (2-3), einem Boden (5) und einem Deckel (6) aus wärmedämmendem Material, einer Wasserleitung (8) zur Kühlung der Innenräume des Kastens, wobei die Austrittsöffnung (11) dieser Wasserleitung mit der Bewässerungsanlage eines Gewächshausdaches oder der Tropfenspeisung eines Gewächshauses verbunden ist, einem an der Wasserleitung angebrachten Absperrhahn (10), mehreren Platten (14) aus wärmedämmendem Material, die den Kasten in mehrere abgeteilte gleichgrosse Fächer (13) unterteilen, in welchen die Nischen (15) für die Drohnen angebracht werden, einer gemeinsamen stirnseitig angebrachten Platte (12) aus wärmedämmendem Material die den Kasten vorderseitig abschliesst, und in der für jedes Fach eine Flugöffnung (16) und Luftlöcher (18) zum Ein- und Auslass der Drohnen bzw. zur Belüftung eingebracht sind, und einer abnehmbaren Wand (19) aus wärmedämmendem Material zum Verschliessen der Rückseite jedes Fachs (13).

2. Kühlschrank nach Anspruch 1, dadurch gekennzeichnet, dass sich in der Innenfläche der Seitenwände (2-3) und im Boden (5) des Kastens Rillen (4-7) befinden, in welchen die Wasserleitung (8) eingebracht ist.

3. Kühlschrank nach Anspruch 1, dadurch gekennzeichnet, dass der Wärmedämmstoff aus Polystyrol und dem sogenannten "Tempex" besteht und dass die Wasserleitung (8) aus flexibelem PVC hergestellt ist.

4. Kühlschrank nach Anspruch 1, dadurch gekennzeichnet, dass der Absperrhahn (20) durch einen im Kasten befindlichen Steuermechanismus mit Thermostat gesteuert wird.

5. Kühlschrank nach Anspruch 1, dadurch gekennzeichnet dass in jeder Flugöffnung (16) ein kleines glattes Schutzrohr (17) angebracht ist, dass die Drohnen beim Hinaus- oder Hineinfliegen vor Verletzungen schützen soll.

6. Kühlschrank nach Anspruch 1, dadurch gekennzeichnet, dass zwischen dem Kasten (1) und jeder abnehmbaren Wand (19) Halterungen für die Befestigung jeder abnehmbaren Wand vorgesehen sind.
